# EUROPEAN PATENT APPLICATION

(11) **EP 2 412 365 A1**
(43) Date of publication of application: **01.02.2012**
(21) Application number: 10755821.5
(22) Date of filing: 03.03.2010
(51) Int. Cl.: A61K 8/895, A61K 8/06, A61K 8/37, A61K 8/44, A61K 8/49, A61Q 17/04

(54) **SUNSCREEN COSMETIC**

(30) Priority: 26.03.2009 JP 2009075345; 27.03.2009 JP 2009079685
(71) Applicant: Shiseido Co., Ltd., Tokyo 104-8010 (JP)
(72) Inventor: ISHITOBI, Sawako, Yokohama-shi Kanagawa 224-8558 (JP); SHIRAO, Masayuki, Yokohama-shi Kanagawa 224-8558 (JP); ABE, Koji, Yokohama-shi Kanagawa 224-8558 (JP); YAMAGUCHI, Kazuhiro, Yokohama-shi Kanagawa 224-8558 (JP)
(74) Representative: ter Meer, Nicolaus
(86) International application number: PCT/JP2010/053444
(87) International publication number: WO 2010/110020

(57) **Abstract**

The present invention provides a sunscreen cosmetic characteristically comprising (1) 2-ethylhexyl p-methoxycinnnamate, (2) Hexyl diethylaminohydroxybenzoylbenzoate, (3) dimethicodiethyl benzal malonate, and (4) 2, 4-bis{[4-(2-ethylhexyloxy)-2-hydroxy]phenyl}-6-(4-methoxyphenyl)-1, 3, 5-triazine.

The present invention also provides a sunscreen cosmetic characteristically comprising
(1) 2-ethylhexyl p-methoxycinnnamate,
(2) Hexyl diethylaminohydroxybenzoylbenzoate,
(3) dimethicodiethyl benzal malonate,
(4) 2, 4-bis{[4-(2-ethylhexyloxy)-2-hydroxy]phenyl}-6-(4-methoxyphenyl)1, 3, 5-triazine, and
(5) powder.

The object of the present invention is to provide a sunscreen cosmetic that gives a superior sensation during use and has a high SPF.

## Description

### TECHNICAL FIELD

The present invention relates to a sunscreen cosmetic. More specifically, it relates to a sunscreen cosmetic that has a superior sensation during use and ultraviolet absorption effect.

### BACKGROUND ART

Important ultraviolet wavelength regions absorbed by sunscreen cosmetics are the UV-A region (320-400 nm) and UV-B region (290-320 nm). It was believed that the ultraviolet light in the UV-A region (320-400 nm) darkened the skin but it would not cause sunburn and accelerate aging of the skin as the ultraviolet light in the UV-B region (290-320 nm) would. However, in recent years, it has been made clear that, whereas the ultraviolet light in the UV-B region only reaches the surface part of the skin, the ultraviolet light in the UV-A region reaches the deeper part of the skin and induces not only skin aging but also skin cancer.

Ultraviolet absorbents for cosmetics that have been used up to the present are structurally categorized into (1) benzoic acid derivatives, (2) cinnamic acid derivatives, (3) benzophenone derivatives, (4) dibenzoylmethane derivatives, and (5) salicylic acid derivatives. In recent years, ultraviolet absorbents of (2) and (4) are frequently used.

However, the ultraviolet absorbents listed above each have problems from a practical point of view. For example, 2-ethylhexyl-p-dimethylaminobenzoate, an example of (1) benzoic acid derivatives, is a transparent liquid and has the advantage of being easy to handle; however, it and its derivatives are questionable in terms of safety and therefore are not used in recent years. Also, its peak absorption wavelength is near 290 nm and it absorbs only the ultraviolet light in the UV-B region.

Among (2) cinnamic acid derivatives, 2-ethylhexyl p-methoxycinnnamate is the most frequently used ultraviolet absorbent in the sun care cosmetics currently available commercially. Its maximum absorption wavelength is near 310 nm and its absorption region does not reach the UV-A region. Also, sunlight degenerates it and therefore it has problems with staining and also with instability of the ultraviolet protection effect.

As for (3) benzophenone derivatives, 2-hydroxy-4-methoxybenzophenone, for example, absorbs both the UV-A and UV-B regions and exhibits relatively good solubility in external preparation base agents; however, its maximum absorption wavelength is rather close to the UV-B region and the absorbance is not very high. Also, in recent years, its basic structural skeleton (benzophenone) has been implicated as an environmental hormone and its use has been avoided.

Among (4) dibenzoylmethane derivatives, 4-tert-buthoxy-4-methoxybenzoylmethane is frequently used for external preparations. Its maximum absorbance is at around 360 nm and the absorbance level is high, and therefore it is a superior ultraviolet absorbent in the UV-A region. However, it has a problem in photostability, and it exhibits poor compatibility with oil components in external preparations and therefore only a small amount can be added.

Among (5) salicylic acid derivatives, octyl salicylate is used. It has a maximum absorption wavelength in the UV-B region, and it is in an oil form and exhibits superior compatibility with paraffin oil and such; however, since its absorbance is low, it is not put to practical use much.

Therefore, 2-ethylhexyl-p-methoxycinnamate from (2) is often used in the UV-B region and 4-tert-butyl-4'-methoxybenzoylmethane from (4) is often used in the UV-A region. In recent years in particular, there is an increasing demand for ultraviolet absorption in the UV-A region.

Some ultraviolet absorbents are sticky, which can be a significant problem when they are blended into sunscreen cosmetics, for which the sensation during use is deemed important. That is, when the blend ratio of the ultraviolet absorbent is increased for the purpose of a higher ultraviolet absorption effect, the sensation during use dramatically worsens. Therefore, there are cases where the desired blend ratio cannot be achieved with the desired ultraviolet absorbent.

Also, generally, a practice of blending multiple ultraviolet absorbents into a sunscreen cosmetic is done for the purpose of securing a broad absorption range.
However, in the case of sunscreen cosmetics containing an ultraviolet absorbent, the sensation during use may degrade. Since a superior sensation during use is an important element strongly required for cosmetics, the practice of blending in multiple ultraviolet absorbents, particularly 3, 4 or more different types, is not usually done, even for the purpose of securing a broad absorption range.

Meanwhile, dibenzoylmethane derivatives, which are blended into cosmetics as ultraviolet absorbents, lose some of their UV absorption capacity when exposed to ultraviolet light. Technology that uses both a benzyolmethane derivative and α-cyano-β , β-diphenylacrylate, which is another ultraviolet absorbent, has been developed in order to control this phenomenon and secure the photostability (Patent Document 1).

Also, in the case of a cosmetic that uses both ultraviolet absorbents, a 1,3,5-triazine derivative and a dibenzoylmethane derivative, the 1,3,5-triazine derivative chemically degrades significantly by ultraviolet irradiation under the presence of 4-tert-butyl-4'-methoxydibenzoylmethane. Therefore, technology that additionally uses α-cyano-β , β-diphenylacrylate to secure photostability of the 1,3,5-triazine derivative and dibenzoylmethane derivative has been developed (Patent Document 2).

However, there is a problem in the technology that secures photostability of the ultraviolet absorbents themselves by using multiple ultraviolet absorbents. Because ultraviolet absorbents have low compatibility with, and are hardly soluble in, the oil components that are the base agent of the cosmetics, therefore, if they are to be stably blended into cosmetics, a large amount of specific oil components having superior compatibility needs to be added. The presence of specific oil components in large quantities would be a cause of a reduction in the stability of the cosmetic (particularly the emulsification stability of water-in-oil emulsified cosmetics) and a reduction in the sensation during use.

As described thus far, it is not a typical practice to add multiple, particularly three, four or more, ultraviolet absorbents to a sunscreen cosmetic.

On the other hand, in addition to an ultraviolet absorbent, the powder of ultraviolet scattering agents such as titanium oxide and zinc oxide are usually added to a sunscreen cosmetic.

### PRIOR ART DOCUMENTS

### PATENT DOCUMENTS

Patent Document 1: Japanese Patent No. 2975682
Patent Document 2: Japanese Patent No. 3714632

### DISCLOSURE OF INVENTION

### TECHNICAL PROBLEM

### (Invention of claims 1-4)

In view of the aforementioned problem, the inventors conducted earnest research on sunscreen cosmetics that have a superior ultraviolet absorption effect and also superior sensation during use, and discovered that a sunscreen cosmetic that manifests a superior ultraviolet absorption effect and has a high SPF, and also provides a excellent sensation during use when an ultraviolet scattering agent powder is not added, could be obtained by blending in a combination of four specific ultraviolet absorbents, thus completing the present invention.

### (Invention of claims 5-10)

Also, in view of the aforementioned problem, the inventors conducted earnest research on sunscreen cosmetics that have a superior ultraviolet absorption effect and also superior sensation during use, and discovered that a sunscreen cosmetic that manifests a superior ultraviolet absorption effect and has a high SPF can be provided by blending in four specific ultraviolet absorbents and the powder of an ultraviolet scattering agent. Furthermore, the inventers also discovered that a sunscreen cosmetic that gives a superior sensation during use can be provided if the powder content is specified, thus completing the present invention.

The object of the present invention is to provide a sunscreen cosmetic that gives a superior sensation during use and also has a high SPF due to a superior ultraviolet absorption effect.

### TECHNICAL SOLUTION

### (Invention of claims 1-4)

That is, the present invention provides a sunscreen cosmetic characteristically comprising
(1) 2-ethylhexyl p-methoxycinnnamate,
(2) Hexyl diethylaminohydroxybenzoylbenzoate,
(3) dimethicodiethyl benzal malonate, and
(4) 2,4-bis{[4-(2-ethylhexyloxy)-2-hydroxy]phenyl}-6-(4-methoxyphenyl)-1,3,5-triazine.

Also, the present invention provides the aforementioned sunscreen cosmetic wherein said sunscreen cosmetic does not contain an ultraviolet scattering agent.

Furthermore, the present invention provides the aforementioned sunscreen cosmetic wherein the blend ratio of said (1) 2-ethylhexyl p-methoxycinnnamate is 1-10 wt%, the blend ratio of said (2) Hexyl diethylaminohydroxybenzoylbenzoate is 0.1-10 wt%, the blend ratio of said (3) dimethicodiethyl benzal malonate is 1-10 wt%, and the blend ratio of said (4) 2,4-bis{[4-(2-ethylhexyloxy)-2-hydroxy]phenyl}-6-(4-methoxyphenyl)-1,3,5-triazine is 0.05-3 wt% of the total amount of the sunscreen cosmetic.

Also, the present invention provides the aforementioned sunscreen cosmetic wherein said sunscreen cosmetic is a water-in-oil emulsified cosmetic.

### (Invention of claims 5-10)

That is, the present invention provides a sunscreen cosmetic characteristically comprising
(1) 2-ethylhexyl p-methoxycinnnamate,
(2) Hexyl diethylaminohydroxybenzoylbenzoate,
(3) dimethicodiethyl benzal malonate,
(4) 2,4-bis{[4-(2-ethylhexyloxy)-2-hydroxy]phenyl}-6-(4-methoxyphenyl)1,3,5-triazine, and
(5) powder.

Also, the present invention provides the aforementioned sunscreen cosmetic wherein said (5) powder is an ultraviolet scattering agent.

Furthermore, the present invention provides the aforementioned sunscreen cosmetic wherein said ultraviolet scattering agent is titanium oxide and/or zinc oxide.

Furthermore, the present invention provides the aforementioned sunscreen cosmetic wherein the blend ratio of said powder is 0.1-5.0 wt% of the total amount of the sunscreen cosmetic.

Furthermore, the present invention provides the aforementioned sunscreen cosmetic wherein the blend ratio of said (1) 2-ethylhexyl p-methoxycinnnamate is 1-10 wt%, the blend ratio of said (2) Hexyl diethylaminohydroxybenzoylbenzoate is 0.1-10 wt%, the blend ratio of said (3) dimethicodiethyl benzal malonate is 1-10 wt%, and the blend ratio of said (4) 2,4-bis{[4-(2-ethylhexyloxy)-2-hydroxy]phenyl}-6-(4-methoxyphenyl)-1,3,5-triazine is 0.05-3 wt% of the total amount of the sunscreen cosmetic.

Also, the present invention provides the aforementioned sunscreen cosmetic wherein said sunscreen cosmetic is a water-in-oil emulsified cosmetic.

### ADVANTAGEOUS EFFECTS

### (Invention of claims 1-4)

The sunscreen cosmetic of the present invention gives a superior sensation during use and manifests a superior ultraviolet absorption effect.

### (Invention of claims 5-10)

The sunscreen cosmetic of the present invention gives a superior sensation during use and manifests a superior ultraviolet absorption effect. In particular, the ultraviolet absorbent of the present invention, even when an ultraviolet scattering agent is blended in to achieve a superior ultraviolet protection effect, causes less of the squeaky sensation characteristic of powders and gives a superior sensation during use avoiding mealiness.

### BRIEF DESCRIPTION 0F DRAWINGS

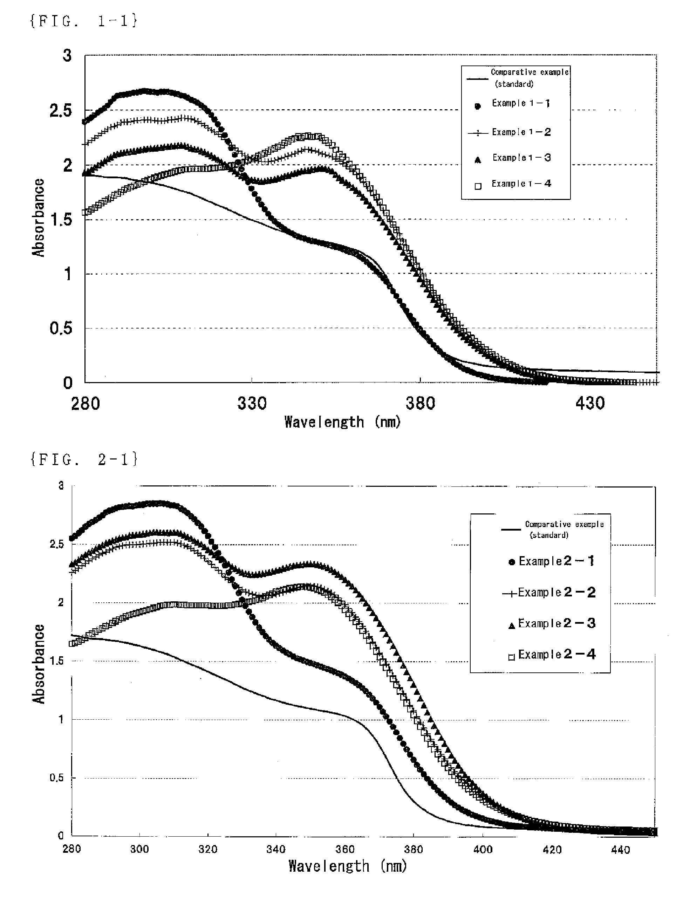

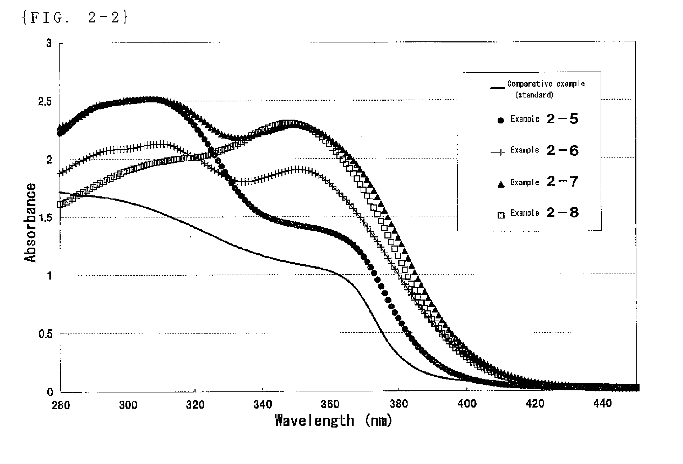

{FIG. 1-1} It shows absorbance curves of Examples and Comparative examples.
{FIG. 2-1} It shows absorbance curves of Examples and Comparative examples.
{FIG. 2-2} It shows absorbance curves of Examples and Comparative examples.

### BEST MODE FOR CARRYING OUT THE INVENTION

The present invention is described in detail below.

(1) 2-ethylhexyl p-methoxycinnnamate
   It is a prior art ultraviolet absorbent and a commercially available form can be used. For example, Parsol MCX (DSM Nutritional Products) is commercially available.
(2) Hexyl diethylaminohydroxybenzoylbenzoate
   It is a prior art ultraviolet absorbent and a commercially available form can be used. For example, Uvinul A Plus (BASF Japan) is commercially available.
(3) Dimethicodiethyl benzal malonate,
   It is a prior art ultraviolet absorbent and a commercially available form can be used. For example, Parsol SLX (DSM Nutritional Products) is commercially available.
(4) 2,4-bis{[4-(2-ethylhexyloxy)-2-hydroxy]phenyl}-6-(4-methoxyphenyl)1,3,5-triazine
   It is a prior art ultraviolet absorbent and a commercially available form can be used. For example, Tinosorb S (Ciba Specialty Chemicals Co., Ltd.) is commercially available.

The blend ratios above are not limited and they are chosen as appropriate; preferable blend ratios are as follows:
The blend ratio of (1) 2-ethylhexyl p-methoxycinnnamate is preferably 1-10 wt%, more preferably 3-10 wt%, of the total amount of the sunscreen cosmetic.
The blend ratio of (2) Hexyl
   diethylaminohydroxybenzoylbenzoate is preferably 0.1-10 wt%, more preferably 1-10 wt%, of the total amount of the sunscreen cosmetic.
The blend ratio of (3) Dimethicodiethyl benzal malonate is preferably 1-10 wt%, more preferably 3-10 wt%, of the total amount of the sunscreen cosmetic.
The blend ratio of (4) 2,4-bis{[4-(2-ethylhexyloxy)-2-hydroxy]phenyl}-6-(4-methoxyphenyl)1,3,5-triazine is preferably 0.05-3 wt%, more preferably 1-3 wt%, of the total amount of the sunscreen cosmetic.

The total blend ratio of the aforementioned four types of the ultraviolet absorbents (1)-(4) are not limited in particular; it is preferably 15-25 wt% of the total amount of the sunscreen cosmetic.
In the present invention, stability in terms of precipitation and odor is maintained even when a large amount of the ultraviolet absorbents is blended in (that is, the stability of the cosmetic as a whole is superior) and therefore the total blend ratio of 8-35 wt% is still preferable. The blend ratio of 4-tert-butyl4'-methoxydibenzoylmethane in Patent Document 1 is limited to 1-5 wt%.

Ultraviolet absorbents other than those described above can also be blended in as appropriate. On the other hand, there is no need to blend in ultraviolet absorbents other than those described above; the aforementioned four types are sufficient as the ultraviolet absorbents to be blended in.

### (Invention of claims 1-4)

It is not necessary to blend an ultraviolet scattering agent into the sunscreen cosmetic of the present invention; it is preferable not to blend it in. The sensation during use improves when an ultraviolet scattering agent is not blended in.

### (Invention of claims 5-10)

The powder used in the present invention is preferably an ultraviolet scattering agent powder. For the ultraviolet scattering agent powder, titanium oxide and/or zinc oxide are preferable. In the present invention, fine particle titanium oxide and/or zinc oxide having an average particle size of 10-100 nm, more preferably 10-50 nm, is preferable. The average particle size is measured with a usual method such as the number average diameter derived from image analysis of transmission electron microscope images.

The blend ratio of the ultraviolet scattering agent powder is preferably 0.1-5.0 wt% of the total weight of the sunscreen cosmetic. If it exceeds 5 wt%, then powderiness and/or dryness (specifically squeakiness and mealiness of the powder) can be felt, leading to undesirable sensations during use. If it is less than 0.1 wt%, then the effect of the ultraviolet scattering agent may be not expected.
The powder in the present invention includes not only the powder as an ultraviolet scattering agent but also usual powder ingredients to adjust usability. For example, it is also preferable to blend in spherical powder (such as PMMA powder and silicone resin powder) and platelike powder (such as talc) as appropriate.

In addition to the aforementioned essential ingredients, other ingredients commonly used in cosmetics can be blended in as necessary in the sunscreen cosmetic of the present invention; examples of such ingredients include whitening agents, humectants, antioxidants, oil-based ingredients, surfactants, thickeners, alcohols, coloring agents, water-based ingredients, water, various skin nutrients, and powder ingredients, and the sunscreen cosmetic can be prepared with a conventional method. The following are some examples of the ingredients.
In the present invention, it is preferable not to blend in an ultraviolet scattering agent ingredient from the point of view of sensation during use.

Oil components such as avocado oil, macadamia nut oil, corn oil, olive oil, rapeseed oil, evening primrose oil, castor oil, sunflower oil, tea seed oil, rice bran oil, jojoba oil, cacao oil, coconut oil, squalene, beef tallow, Japanese core wax, beeswax, candelilla wax, carnauba wax, whale wax, lanolin, liquid paraffin, polyoxyethylene (8 mole) oleyl alcohol ether, glyceryl monooleate, cyclomethicone, dimethylpolysiloxane, and diphenylpolysiloxane.

Higher alcohols such as caprylic alcohol, lauryl alcohol, myristyl alcohol, cetyl alcohol, cholesterol, and phytosterol.

Higher fatty acids such as caprylic acid, lauric acid, myristic acid, palmitic acid, stearic acid, behenic acid, lanolin fatty acid, linoleic acid, and linolenic acid.

Humectants such as polyethylene glycol, glycerin, sorbitol, xylitol, maltitol, mucopolysaccharide, hyaluronic acid, chondroitin sulfate, and chitosan.

Thickeners such as methyl cellulose, ethyl cellulose, Arabic gum, and polyvinyl alcohol.

Organic solvents such as ethanol and 1,3-butylene glycol.

Antioxidants such as butylhydroxytoluene, tocopherol, and phytic acid.

Antibacterial preservatives such as benzoic acid, salicylic acid, sorbic acid, paraoxybenzoic esters (ethylparaben and butylparaben, for example), and hexachlorophene.
Amino acids such as glycine, alanine, valine, leucine, serine, threonine, phenyalanine, tyrosine, aspartic acid, asparagine, glutamine, taurine, arginine, and histidine, as well as hydrochlorides thereof.
Organic acids such as acyl sarcosinic acid (sodium lauroyl sarcosinate, for example), glutathione, citric acid, malic acid, tartaric acid, and lactic acid.
Vitamins such as vitamin A and its derivatives, vitamin B's including vitamin B6 hydrochloride, vitamin B6 tripalmitate, vitamin B6 dioctanoate, vitamin B2 and its derivatives, vitamin B12, and vitamin B15 and its derivatives, vitamin C's including ascorbic acid, ascorbic malic esters (salts), and ascorbic dipalmitate, vitamin E's including α-tocopherol, β-tocopherol, γ-tocopherol, vitamin E acetate, and vitamin E nicotinate, vitamin D's, vitamin H, pantothenic acid, and pantethine.
Various drugs such as nicotinamide, benzyl nicotinate, γ-oryzanol, allantoin, glycyrrhizic acid (salt), glycyrrhizic acid and its derivatives, hinokitiol, musidine, bisabolol, eucalyptol, thymol, inositol, saponins (saikosaponin, carrot saponin, gourd saponin, soapberry saponin, etc.), pantothenylethyl ether, ethynylestradiol, tranexamic acid, cepharanthine, and placenta extract.
Natural extracts from Rumex japonicus, Sophora flavescens, Nuphar japonica, orange, sage, thyme, yarrow, mallow, smilax, swertia, Ligusticum acutilobum, bitter orange peel, birch, horsetail, gourd, horse chestnut, creeping saxifrage, arnica, lily, mugwort, Paeonia lactiflora, aloe, gardenia, Chamaecyparis pisifera, etc. extracted by using an organic solvent, alcohol, polyhydric alcohol, water, hydroalcohol, etc.
Cation surfactants such as stearyltrimethylammonium chloride, benzalkonium chloride, and lauryl amine oxide.
Sequestering agents such as disodium edetate, trisodium edetate, sodium citrate, sodium polyphosphate, sodium metaphosphate, and gluconic acid.
Silicone resin powder, perfumes, scrubbing agents, purified water, etc.

Particularly preferable base agents for the sunscreen cosmetic of the present invention are oil components including decamethylcyclopentasiloxane, isononyl isononanoate, dimethylpolysiloxane, heptamethyloctyltrisiloxane, trimethylsiloxysilicic acid, liquid paraffin, squalane, cetyl isooctanoate, triglyceride octanoate, and di-2-ethylhexyl succinate. It is preferable to blend in one, two or more of these oil components along with the oil component represented by formula (I). The present invention is used preferably in sunscreen cosmetics that use decamethylcyclopentasiloxane as the main base agent.

The sunscreen cosmetic of the present invention is preferably a water-in-oil emulsified cosmetic (W/O emulsion) and it is preferably used as a sunscreen cream, sunscreen emulsion and sunscreen lotion.

### EXAMPLES

The invention is described in specific detail through Examples. The present invention is not limited to these Examples.
The blend ratios are in mass-percentage units unless specified otherwise.

### (Invention of claims 1-4)

The sunscreen cosmetics of Examples and Comparative examples (standards) were prepared with an ordinary method and the sensation during use and the ultraviolet absorption effect were evaluated.

**{Table 1-1}**

| | Example 1-1 | Example 1-2 | Example 1-3 | Example 1-4 |
|---|---|---|---|---|
| Decamethyl cyclopentasi loxane | Balance | Balance | Balance | Balance |
| Dimethyplolysi loxane | 1 | 1 | 1 | 1 |
| Laury PEG-9 polydimethylsiloxyethyl dimethicone | 0.6 | 0.6 | 0.6 | 0.6 |
| Polybutylene glyool/PPG-9/1 copolymer | 2 | 2 | 2 | 2 |
| Diisopropyl sebacate | 5 | 5 | 5 | 5 |
| Ethylhexyl ethylhexanoate | 5 | 5 | 5 | 5 |
| 2-thylhexyl p-methoxycimnamate | 10 | 10 | 10 | 1 |
| Dimethicodiethyl benzal malonate | 10 | 10 | 1 | 10 |
| Hexyl diethy laminohydroxybenzoylbenzoate | 0.1 | 10 | 10 | 10 |
| 2,4-bis[[4-(2-thylhexyloxy)-2-hydroxy]phenyl}-6-(4-methoxyphenyl)1,3,5-triazine | 3 | 0.05 | 3 | 3 |
| (Vinyl dimethicone/methicone silsesquioxane) crosspolymer | 5 | 5 | 5 | 5 |
| Ion-exchanged water | 13 | 13 | 13 | 13 |
| EDTA-3Na | 0.05 | 0.05 | 0.05 | 0.05 |
| Glycerin | 3 | 3 | 3 | 3 |
| Polyoxyethylene methylglycoside | 0.1 | 0.1 | 0.1 | 0.1 |
| Ethanol | 6 | 6 | 6 | 6 |
| Total | 100 | 100 | 100 | 100 |

**{Table 1-2}**

| | Comparative example (standard) |
|---|---|
| Decamethylcyclopentasi loxane | Balance |
| Olefin oligomer | 5 |
| Lauryl PEG-9 polydimethylsi loxyethyl dimethicone | 1 |
| Dimathylpolysi loxane | 5 |
| Isostearic acid | 1 |
| Hydrophobicized zinc oxide (powder) | 15 |
| Hydrophobicized titanium oxide (powder) | 10 |
| Silica | 5 |
| Ion-exchanged water | 20 |
| EDTA-3Na | 0.05 |
| Glycerin | 5 |
| 1,3 butylene glycol | 4 |
| Phenoxy ethanol | 0.5 |
| Total | 100 |

### <Sensation during use>

Actual use tests were conducted by a panel of specialists for evaluation. The results and the evaluation criteria are shown below. The Examples of the present invention all give a superior sensation during use.

### Evaluation criteria

**{Table 1-3}**

| Item | Poor | Evaluation score (5 steps) | Good |
|---|---|---|---|
| Spreadability | Heavy | -2, -1, 0, 1, 2 | Light |
| Powdery squeakiness | Present | 2,-1,0,1,2 | Not present |
| Mealiness | Present | -2, -1, 0, 1, 2 | Not present |
| Dryness | Present | -2, -1 ,0, 1, 2 | Not present |
| Smoothness | Not present | -2, -1, 0, 1, 2 | Present |
| Filminess | Present | 2, -1, 0, 1, 2 | Not present |
| Coating color | White | 2, -1, 0, 1, 2 | Not white |
| General preference | Dislike | 2,-1, 0,1, 2 | Like |

### Evaluation results of Example 1-1 using Comparative example (standard) as zero

**{Table 1-4}**

| Item | Panel A | Panel B | Panel C | Panel D | Panel E | Panel F | Panel G | Panel H | Average |
|---|---|---|---|---|---|---|---|---|---|
| Spreadability | 1 | 1 | 1 | 2 | 0 | 1 | 1 | 2 | 1.125 |
| Powdery squeakiness | 1 | 2 | 1 | 2 | 2 | 2 | 1 | 2 | 1.625 |
| Mealiness | 1 | 1 | 2 | 1 | 0 | 1 | 2 | 1 | 1.125 |
| Dryness | 1 | 1 | 2 | 0 | 2 | 1 | 0 | 2 | 1.125 |
| Smoothness | 1 | 1 | 2 | 2 | 1 | 1 | 2 | 2 | 1.5 |
| Filminess | 1 | 2 | 1 | 1 | 2 | 2 | 1 | 1 | 1.375 |
| Coating color | 2 | 2 | 2 | 2 | 1 | 2 | 2 | 2 | 1.875 |
| General preference | 1 | 2 | 1 | 1 | 0 | 2 | 1 | 1 | 1.125 |

### Evaluation results of Example 1-2 using Comparative example (standard) as zero

**{Table 1-5}**

| Item | Panel A | Panel B | Panel C | Panel D | Panel E | Panel F | Panel G | Panel H | Average |
|---|---|---|---|---|---|---|---|---|---|
| Spreadability | 2 | 1 | 1 | 2 | 0 | 1 | 1 | 2 | 1.25 |
| Powdery squeakiness | 1 | 2 | 1 | 2 | 2 | 2 | 1 | 2 | 1.625 |
| Mealiness | 2 | 1 | 2 | 1 | 0 | 1 | 2 | 1 | 1.25 |
| Dryness | 0 | 1 | 2 | 0 | 2 | 1 | 0 | 2 | 1 |
| Smoothness | 1 | 1 | 2 | 2 | 1 | 1 | 2 | 2 | 1.5 |
| Filminess | 2 | 2 | 1 | 1 | 2 | 2 | 1 | 1 | 1.5 |
| Coating color | 2 | 2 | 2 | 2 | 2 | 1 | 2 | 2 | 1.875 |
| General preference | 1 | 2 | 1 | 1 | 1 | 2 | 1 | 1 | 1.25 |

### Evaluation results of Example 1-3 using Comparative example (standard) as zero

**{Table 1-6}**

| Item | Panel A | Panel B | Panel C | Panel D | Panel E | Panel F | Panel G | Panel H | Average |
|---|---|---|---|---|---|---|---|---|---|
| Spreadability | 1 | 1 | 1 | 2 | 0 | 2 | 1 | 2 | 1.25 |
| Powdery squeakiness | 1 | 2 | 1 | 2 | 2 | 2 | 1 | 2 | 1.625 |
| Mealiness | 1 | 1 | 2 | 1 | 0 | 1 | 1 | 1 | 1 |
| Dryness | 1 | 1 | 2 | 0 | 2 | 0 | 0 | 2 | 1 |
| Smoothness | 1 | 1 | 2 | 2 | 1 | 1 | 2 | 2 | 1.5 |
| Filminess | 1 | 2 | 1 | 1 | 2 | 2 | 1 | 1 | 1.375 |
| Coating color | 2 | 2 | 2 | 2 | 2 | 2 | 2 | 2 | 2 |
| General preference | 1 | 2 | 1 | 1 | 1 | 2 | 1 | 1 | 1.25 |

### Evaluation results of Example 1-4 using Comparative example (standard) as zero

**{Table 1-7}**

| Item | Panel A | Panel B | Panel C | Panel D | Panel E | Panel F | Panel G | Panel H | Average |
|---|---|---|---|---|---|---|---|---|---|
| Spreadability | 1 | 1 | 1 | 2 | 0 | 1 | 1 | 1 | 1 |
| Powdery squeakiness | 1 | 1 | 1 | 2 | 1 | 2 | 1 | 2 | 1.375 |
| Mealiness | 1 | 1 | 2 | 1 | 1 | 1 | 2 | 1 | 1. 25 |
| Dryness | 1 | 0 | 2 | 0 | 0 | 1 | 0 | 2 | 0.75 |
| Smoothness | 1 | 1 | 2 | 1 | 1 | 1 | 2 | 2 | 1.375 |
| Filminess | 1 | 1 | 1 | 0 | 0 | 1 | 0 | 1 | 0.625 |
| Coating color | 2 | 2 | 2 | 2 | 2 | 2 | 2 | 2 | 2 |
| General preference | 1 | 1 | 1 | 1 | 1 | 2 | 1 | 1 | 1.125 |

### <Ultraviolet absorption effect>

50 µL of samples from Examples and Comparative example were applied uniformly on a nylon membrane (5 x 5 cm) at a density of 2 mg/cm²; after this was allowed to stand for 15 minutes, a spectrophotometer (U-4100 from Hitachi, Ltd.) was used to measure the absorbance.
The results shown in FIG. 1-1 indicate that the UV absorption of Examples is far superior to that of Comparative example. The SPF (Sun Protection Factor ) and PA (Protection Grade of UVA) of the Comparative example are 34 and +++, respectively; therefore, the UV absorption of Examples is SPF of 30 or higher and a PA of +++ or higher, which confirms the fact that they are ultraviolet protection cosmetics having a high SPF and PA.

The following are examples of the sunscreen cosmetic of the present invention. Each of them is a sunscreen cosmetic having a superior sensation during use and ultraviolet absorption, as well as superior emulsification stability.

| Example 1-5: Sunscreen emulsion (W/O) | | wt% |
|---|---|---|
| (1) | Decamethylcyclopentasiloxane | 20 |
| (2) | Polyoxyethylene-methylpolysiloxane copolymer | 1 |
| (3) | Olefin oligomer | 10 |
| (4) | Dimethylpolysiloxane | 5 |
| (5) | 2-ethylhexyl p-methoxycinnnamate | 10 |
| (6) | Dimethicodiethyl benzal malonate | 5 |
| (7) | Hexyl diethylaminohydroxybenzoylbenzoate | 3 |
| (8) | Perfume | 0.5 |
| (9) | 2,4-bis {[4-(2-ethylhexyloxy)-2-hydroxy] phenyl}-6-(4-methoxyphenyl)-1,3,5-triazine | 2 |
| (10) | (Vinyl dimethicone/methicone silsesquioxane) crosspolymer | 5 |
| (11) | 1,3-butylene glycol | 5 |
| (12) | Ion-exchanged water | Balance |
| (13) | Glycerin | 3 |
| (14) | Edetate | Appropriate amount |

Preparation method:
(1)-(9) are mixed and dissolved at room temperature to prepare the oil phase beforehand. (10) is gradually added to the oil phase as it is stirred with a disper. (11)-(14) are mixed and dissolved and then gradually added to the oil phase as they are stirred with a disper, followed by thorough mixing and dissolution to obtain the target sunscreen emulsion.

| Example 1-6: Sunscreen emulsion (W/O) | | wt% |
|---|---|---|
| (1) | Decamethylcyclopentasiloxane | 20 |
| (2) | Polyoxyethylene-methylpolysiloxane copolymer | 1 |
| (3) | Olefin oligomer | 10 |
| (4) | Dimethylpolysiloxane | 10 |
| (5) | 2-ethylhexyl p-methoxycinnnamate | 10 |
| (6) | Hexyl diethylaminohydroxybenzoylbenzoate | 5 |
| (7) | 2,4-bis {[4-(2-ethylhexyloxy)-2-hydroxy] phenyl}-6-(4-methoxyphenyl)-1,3,5-triazine | 0. 5 |
| (8) | Perfume | 0.5 |
| (9) | Dimethicodiethyl benzal malonate | 0.1 |
| (10) | Paraben | 5 |
| (11) | Ion-exchanged water | Balance |
| (12) | Glycerin | 3 |
| (13) | Edetate | Appropriate amount |

Preparation method:
(1)-(9) are mixed and dissolved at room temperature to prepare the oil phase beforehand. (10)-(14) are mixed and dissolved and then gradually added to the oil phase as they are stirred with a disper, followed by thorough mixing and dissolution to obtain the target sunscreen emulsion.

| Example 1-7: Sunscreen emulsion (O/W) | | wt% |
|---|---|---|
| (1) | Carboxyvinyl polymer | 0.3 |
| (2) | Purified water | Balance |
| (3) | Xanthan gum | 0.1 |
| (4) | Trisodium edentate | 0.1 |
| (5) | Dipotassium glycyrrhizate | 0.05 |
| (6) | 1,3-butylene glycol | 5 |
| (7) | Polyoxyethylene glyceryl isostearate | 1.5 |
| (8) | Polyoxyethylene glycerol monostearate | 1 |
| (9) | Ascorbic acid glucoside | 2 |
| (10) | Phenoxyethanol | Appropriate amount |
| (11) | Decamethylcyclopentasiloxane | 3 |
| (12) | Methylphenylsiloxane | 3 |
| (13) | Behenyl alcohol | 1 |
| (14) | 2-ethylhexyl p-methoxycinnnamate | 7 |
| (15) | Hexyl diethylaminohydroxybenzoylbenzoate | 2 |
| (16) | 2,4-bis ([4-(2-ethylhexyloxy)-2-hydroxy] phenyl)-6-(4-methoxyphenyl)-1,3,5-triazine | 2 |
| (17) | Dimethicodiethyl benzal malonate | 5 |
| (18) | Perfume | Appropriate amount |

Preparation method:
(1)-(10) are heated up to 70° C, mixed, and dissolved to prepare the water phase. (11)-(18) are heated, mixed, and dissolved to prepare the oil phase, which is then gradually added to the water phase as it is stirred with a disper, followed by thorough mixing and dissolution to obtain the target sunscreen emulsion.

| Example 1-8: Sunscreen cream (W/O) | | wt% |
|---|---|---|
| (1) | Decamethylcyclopentasiloxane | 20 |
| (2) | Polyoxyethylene/methylpolysiloxane copolymer | 3 |
| (3) | Cetyl isooctanoate | 10 |
| (4) | Dimethylpolysiloxane | 5 |
| (5) | 2-ethylhexyl p-methoxycinnnamate | 5 |
| (6) | Hexyl diethylaminohydroxybenzoylbenzoate | 1 |
| (7) | 2,4-bis {[4-(2-ethylhexyloxy)-2-hydroxy] phenyl}-6-(4-methoxyphenyl)-1,3,5-triazine | 3 |
| (8) | Dimethicodiethyl benzal malonate | 3 |
| (9) | Octocrylene | 3 |
| (10) | Distearyl ammonium chloride | 0.001 |
| (11) | Phenoxy ethanol | 0.5 |
| (12) | Perfume | 0.5 |
| (13) | Organic modified clay mineral | 2.5 |
| (14) | Silica powder | 6 |
| (15) | Ion-exchanged water | Balance |
| (16) | 1,3-butylene glycol | 2 |
| (17) | Edetate | Appropriate amount |

Preparation method:
(1)-(12) are heated up to 70° C, mixed and dissolved to prepare the oil phase beforehand. (13)-(14) are added and dispersed and mixed with a disper. (15)-(17) are mixed and dissolved and then gradually added to the oil phase as they are stirred with a disper, followed by thorough mixing and dissolution to obtain the target sunscreen cream.

### (Invention of claims 5-10)

Sunscreen cosmetics of Examples and Comparative examples (standards) were prepared with an ordinary method and the sensation during use and the ultraviolet absorption effect were evaluated.

**{Table 2-1}**

| | Example 2-1 | Example 2-2 | Example 2-3 | Exanple 2-4 |
|---|---|---|---|---|
| Decamethylcyclopentasiloxane | Balance | Balance | Balance | Balance |
| Dimethylpolysiloxane | 1 | 1 | 1 | 1 |
| Lauryl PEG-9 polydimethylsiloxyethyl dimethicone | 0.6 | 0.6 | 0.6 | 0.6 |
| Polybutylene glycol/PPG-9/1 copolymer | 2 | 2 | 2 | 2 |
| Diisopropyl sebacate | 5 | 5 | 5 | 5 |
| Ethylhexyl ethylhexanoate | 5 | 5 | 5 | 5 |
| 2-ethylhexyl p-methoxycinnnamate, | 10 | 10 | 10 | 0.5 |
| Dimethicodiethyl benzal malonate | 10 | 10 | 0.5 | 10 |
| Hexyl diethylaminohydroxybenzoylbenzoate | 0.1 | 10 | 10 | 10 |
| 2,4-bis{[4-(2-ethylhexyloxy)-2-hydroxy]phenyl}-6-(4-methoxyphenyl)1,3,5-triazine | 3 | 0.01 | 3 | 3 |
| Hydrophobicized fine particle titanium oxide (powder) | 5 | 5 | 5 | 5 |
| Hydrophobicized fine particle zinc oxide (powder) | - | - | - | - |
| (Vinyl dimethicone/methicone silsesquioxane) crosspolymer | 5 | 5 | 5 | 5 |
| Ion-exchanged water | 13 | 13 | 13 | 13 |
| EDTA-3Na | 0.05 | 0.05 | 0.05 | 0.05 |
| Glycerin | 3 | 3 | 3 | 3 |
| Polyoxyethylene methylglycoside | 0.1 | 0.1 | 0.1 | 0.1 |
| Ethanol | 6 | 6 | 6 | 6 |
| Total | 100 | 100 | 100 | 100 |

**{Table 2-2}**

| | Example 2-5 | Example 2-6 | Example 2-7 | Example 2-8 |
|---|---|---|---|---|
| Decamiethylcyciopentasiloxane | Balance | Balance | Balance | Balance |
| Dimethylpolysiloxane | 1 | 1 | 1 | 1 |
| Lauryl PEG-9 polydimethylsiloxyethyl dimethicone | 0.6 | 0.6 | 0.6 | 0.6 |
| Polybutylene glycol/PPG-9/1 copolymer | 2 | 2 | 2 | 2 |
| Diisopropyl sebacate | 5 | 5 | 5 | 5 |
| Ethylhexyl ethylhexanoate | 5 | 5 | 5 | 5 |
| 2-ethylhexyl p-methoxycinnnamate, | 10 | 10 | 10 | 0.5 |
| Dimethicodiethyl benzal malonate, | 10 | 10 | 0.5 | 10 |
| Hexyl diethylaminohydroxybenzoylbenzoate | 0.1 | 10 | 10 | 10 |
| 2,4-bis{[4-(2-ethylhexyloxy)-2-hydroxy]phenyl}-6-(4-methoxyphenyl) 1, 3, 5-triazine | 3 | 0.01 | 3 | 3 |
| Hydrophobicized fine particle titanium oxide (powder) | - | - | - | - |
| Hydrophobicized fine particle zinc oxide (powder) | 5 | 5 | 5 | 5 |
| (Vinyl dimthicone/methicone silsesquioxane) crosspolymer | 5 | 5 | 5 | 5 |
| Ion-exchanged water | 13 | 13 | 13 | 13 |
| EDTA-3Na | 0.05 | 0.05 | 0.05 | 0.05 |
| Glycerin | 3 | 3 | 3 | 3 |
| Polyoxyethylene methylglycoside | 0.1 | 0.1 | 0.1 | 0.1 |
| Ethanol | 6 | 6 | 6 | 6 |
| Total | 100 | 100 | 100 | 100 |

**{Table 2-3}**

| | Comparative example (standard) |
|---|---|
| Decamthylcyclopentasiloxane | 28.45 |
| Olefin oligcmr | 5 |
| Lauryl PEG-9 polydimethylsiloxyethyl dimethicone | 1 |
| Dimthylpolysiloxane | 5 |
| Isostearic acid | 1 |
| Hydrophobicized zinc oxide (powder) | 15 |
| Hydrophobicized titanium oxide (powder) | 10 |
| Silica | 5 |
| Ion-exchanged water | 20 |
| EDTA-3Na | 0.05 |
| Glycerin | 5 |
| 1,3-butylene glycol | 4 |
| Phenoxy ethanol | 0.5 |
| Total | 100 |

### <Sensation during use>

Actual use tests were conducted by a panel of specialists for evaluation. The results and the evaluation criteria are shown below. The Examples of the present invention all give a superior sensation during use.

### Evaluation criteria

**{Table 2-4}**

| Item | Poor | Evaluation score (5 steps) | Good |
|---|---|---|---|
| Spreadability | Heavy | -2, -1, 0, 1, 2 | Light |
| Powdery squeakiness | Present | -2,-1,0,1,2 | Not present |
| Mealiness | Present | -2, -1, 0, 1, 2 | Not present |
| Dryness | Present | -2, -1, 0, 1, 2 | Not present |
| Smoothness | Not present | -2, -1, 0, 1, 2 | Present |
| Filminm | Present | -2, -1, 0, 1, 2 | Not present |
| Coating color | White | -2, -1, 0, 1, 2 | Not white |
| General preference | Dislike | 2, -1, 0, 1, 2 | Like |

### Evaluation results of Example 2-1 using Comparative example (standard) as zero

**{Table 2-5}**

| Item | Panel A | Panel B | Panel C | Panel D | Panel E | Panel F | Panel G | Panel H | Average |
|---|---|---|---|---|---|---|---|---|---|
| Spreadability | 1 | 1 | 1 | 1 | 0 | 2 | 1 | 2 | 1.125 |
| Powdery squeakiness | 1 | 2 | 2 | 1 | 1 | 2 | 1 | 2 | 1.5 |
| Meahness | 1 | 1 | 2 | 1 | 0 | 2 | 2 | 1 | 1. 25 |
| Dryness | 0 | 1 | 2 | 0 | 1 | 1 | 0 | 1 | 0.75 |
| Smoothness | 1 | 1 | 2 | 1 | 1 | 1 | 2 | 2 | 1.375 |
| Filminess | 1 | 2 | 1 | 1 | 0 | 2 | 1 | 1 | 1.125 |
| Coating color | 1 | 1 | 1 | 1 | 1 | 2 | 1 | 2 | 1.25 |
| General preference | 1 | 1 | 2 | 1 | 0 | 2 | 1 | 1 | 1.125 |

### Evaluation results of Example 2-2 using Comparative example (standard) as zero

**{Table 2-6}**

| Item | Panel A | Panel B | Panel C | Panel D | Panel E | Panel F | Panel G | Panel H | Average |
|---|---|---|---|---|---|---|---|---|---|
| Spreadability | 1 | 1 | 1 | 1 | 0 | 2 | 1 | 2 | 1.125 |
| Powdery squeakiness | 0 | 1 | 2 | 1 | 1 | 2 | 1 | 2 | 1. 25 |
| Mealiness | 1 | 0 | 2 | 1 | 0 | 2 | 2 | 1 | 1.125 |
| Dryness | 1 | 1 | 1 | 1 | 0 | 1 | 0 | 1 | 0.75 |
| Smoothness | 1 | 2 | 2 | 1 | 1 | 2 | 2 | 2 | 1.625 |
| Filminess | 1 | 1 | 1 | 1 | 0 | 2 | 1 | 1 | 1 |
| Coating color | 2 | 1 | 1 | 1 | 1 | 2 | 1 | 2 | 1.375 |
| General preference | 1 | 2 | 2 | 1 | -1 | 2 | 2 | 1 | 1.25 |

### Evaluation results of Example 2-3 using Comparative example (standard) as zero

**{Table 2-7}**

| Item | Panel A | Panel B | Panel C | Panel D | Panel E | Panel F | Panel G | Panel H | Average |
|---|---|---|---|---|---|---|---|---|---|
| Spreadability | 1 | 2 | 1 | 1 | 0 | 2 | 1 | 2 | 1.25 |
| Popery squeakiness | 1 | 1 | 2 | 1 | 1 | 2 | 1 | 2 | 1.375 |
| Mealiness | 2 | 0 | 2 | 1 | 0 | 2 | 2 | 1 | 1.25 |
| Dryness | 1 | 1 | 1 | 1 | 1 | 1 | 0 | 1 | 0.875 |
| Smothness | 0 | 1 | 2 | 1 | 0 | 2 | 2 | 2 | 1.25 |
| Filminess | 1 | 2 | 1 | 1 | 0 | 2 | 1 | 1 | 1.125 |
| Coating color | 2 | 1 | 1 | 1 | 1 | 2 | 1 | 2 | 1.375 |
| General preference | 1 | 2 | 2 | 1 | 0 | 2 | 2 | 1 | 1. 375 |

### Evaluation results of Example 2-4 using Comparative example (standard) as zero

**{Table 2-8}**

| Item | Panel A | Panel B | Panel C | Panel D | Panel E | Panel F | Panel G | Panel H | Average |
|---|---|---|---|---|---|---|---|---|---|
| Spreadability | 0 | 1 | 1 | 1 | 0 | 2 | 1 | 2 | 1 |
| Powdery squeakiness | 1 | 2 | 2 | 1 | 1 | 2 | 1 | 2 | 1.5 |
| Mealiness | 2 | 1 | 2 | 1 | 0 | 2 | 2 | 1 | 1.375 |
| Dryness | 1 | 1 | 1 | 1 | 1 | 1 | 0 | 1 | 0.875 |
| Smoothness | 1 | 1 | 2 | 1 | 1 | 1 | 2 | 2 | 1.375 |
| Filminess | 2 | 1 | 1 | 1 | 0 | 2 | 1 | 1 | 1.125 |
| Coating color | 2 | 1 | 1 | 1 | 1 | 2 | 1 | 2 | 1.375 |
| General preference | 1 | 1 | 1 | 1 | -1 | 2 | 2 | 1 | 1 |

### Evaluation results of Example 2-5 using Comparative example (standard) as zero

**{Table 2-9}**

| Item | Panel A | Panel B | Panel C | Panel D | Panel E | Panel F | Panel G | Panel H | Average |
|---|---|---|---|---|---|---|---|---|---|
| Spreadability | 1 | 2 | 1 | 1 | 0 | 2 | 1 | 2 | 1. 25 |
| Powdery squeakiness | 1 | 2 | 2 | 1 | 1 | 2 | 1 | 2 | 1.5 |
| Mealiness | 1 | 1 | 2 | 1 | 0 | 2 | 2 | 1 | 1.25 |
| Dryness | 1 | 1 | 2 | 0 | 0 | 1 | 0 | 1 | 0.75 |
| Smoothness | 1 | 1 | 2 | 1 | 1 | 1 | 2 | 2 | 1.375 |
| Filminess | 1 | 2 | 1 | 1 | 0 | 2 | 1 | 1 | 1.125 |
| Coating color | 1 | 2 | 1 | 2 | 1 | 1 | 2 | 2 | 1.5 |
| General preference | 1 | 1 | 1 | 1 | 0 | 2 | 1 | 1 | 1 |

### Evaluation results of Example 2-6 using Comparative example (standard) as zero

**{Table 2-10}**

| Item | Panel A | Panel B | Panel C | Panel D | Panel E | Panel F | Panel G | Panel H | Average |
|---|---|---|---|---|---|---|---|---|---|
| Spreadability | 2 | 2 | 1 | 1 | 0 | 1 | 1 | 2 | 1.25 |
| Powdery squeakiness | 1 | 2 | 2 | 1 | 1 | 2 | 1 | 2 | 1.5 |
| Mealiness | 1 | 2 | 2 | 1 | 0 | 2 | 2 | 2 | 1.5 |
| Dryness | 1 | 1 | 2 | 0 | 0 | 1 | 0 | 1 | 0.75 |
| Smoothness | 1 | 1 | 2 | 1 | 1 | 1 | 2 | 2 | 1.375 |
| Filminess | 1 | 2 | 0 | 1 | 0 | 2 | 1 | 1 | 1 |
| Coating color | 1 | 1 | 1 | 2 | 1 | 1 | 2 | 2 | 1.375 |
| General preference | 1 | 1 | 1 | 1 | 0 | 2 | 1 | 1 | 1 |

### Evaluation results of Example 2-7 using Comparative example (standard) as zero

**{Table 2-11}**

| Item | Panel A | Panel B | Panel C | Panel D | Panel E | Panel F | Panel G | Panel H | Average |
|---|---|---|---|---|---|---|---|---|---|
| Spreadability | 1 | 2 | 1 | 1 | 0 | 1 | 1 | 2 | 1.125 |
| Powdery squeakiness | 1 | 2 | 2 | 1 | 1 | 2 | 1 | 2 | 1.5 |
| Mealiness | 1 | 1 | 2 | 1 | 0 | 2 | 2 | 2 | 1.375 |
| Dryness | 1 | 1 | 2 | 0 | 0 | 1 | 0 | 1 | 0.75 |
| Smoothness | 1 | 1 | 2 | 1 | 1 | 1 | 2 | 2 | 1.375 |
| Filminess | 1 | 2 | 0 | 1 | 0 | 2 | 1 | 1 | 1 |
| Coating color | 1 | 1 | 1 | 2 | 1 | 1 | 2 | 2 | 1.375 |
| General preference | 1 | 1 | 1 | 1 | 0 | 2 | 1 | 1 | 1 |

### Evaluation results of Example 2-8 using Comparative example (standard) as zero

**{Table 2-12}**

| Item | Panel A | Panel B | Panel C | Panel D | Panel E | Panel F | Panel G | Panel H | Average |
|---|---|---|---|---|---|---|---|---|---|
| Spreadability | 1 | 2 | 1 | 1 | 0 | 1 | 1 | 2 | 1.125 |
| Powdery squeakiness | 1 | 1 | 2 | 1 | 1 | 2 | 1 | 2 | 1.375 |
| Mealiness | 1 | 1 | 2 | 1 | 0 | 2 | 2 | 2 | 1.375 |
| Dryness | 1 | 1 | 2 | 0 | 0 | 1 | 0 | 2 | 0.875 |
| Smoothness | 1 | 1 | 2 | 1 | 1 | 1 | 2 | 2 | 1.375 |
| Filminess | 1 | 2 | 0 | 1 | 0 | 2 | 1 | 1 | 1 |
| Coating color | 1 | 1 | 1 | 2 | 1 | 1 | 2 | 2 | 1. 375 |
| General preference | 1 | 1 | 1 | 1 | 0 | 2 | 1 | 1 | 1 |

### <Ultraviolet absorption effect>

50 µL of samples from Examples and Comparative example were applied uniformly on a nylon membrane (5 x 5 cm) at a density of 2 mg/cm²; after this was allowed to stand for 15 minutes, a spectrophotometer (U-4100 from Hitachi, Ltd.) was used to measure the absorbance.
The results shown in FIG. 2-1 and FIG. 2-2 indicate that the UV absorption of Examples is far superior to that of Comparative example. The SPF (Sun Protection Factor ) and PA (Protection Grade of UVA) of the Comparative example are 34 and +++, respectively; therefore, the UV absorption of Examples is SPF of 30 or higher and a PA of +++ or higher, which confirms the fact that they are ultraviolet protection cosmetics having a high SPF and PA.

The following are examples of the sunscreen cosmetic of the present invention. Each of them is a sunscreen cosmetic having a superior sensation during use and ultraviolet absorption, as well as superior emulsification stability.

| Example 2-9: Sunscreen emulsion (W/O) | | wt% |
|---|---|---|
| (1) | Decamethylcyclopentasiloxane | 20 |
| (2) | Polyoxyethylene-methylpolysiloxane copolymer | 1 |
| (3) | Olefin oligomer | 10 |
| (4) | Dimethylpolysiloxane | 5 |
| (5) | 2-ethylhexyl p-methoxycinnnamate. | 10 |
| (6) | Dimethicodiethyl benzal malonate | 5 |
| (7) | Hexyl diethylaminohydroxybenzoylbenzoate | 3 |
| (8) | Perfume | 0.5 |
| (9) | 2,4-bis {[4-(2-ethylhexyloxy)-2-hydroxy] phenyl}-6-(4-methoxyphenyl)-1,3,5-triazine | 2 |
| (10) | Hydrophobicized zinc oxide | 5 |
| (11) | 1,3-butylene glycol | 5 |
| (12) | Ion-exchanged water | Balance |
| (13) | Glycerin | 3 |
| (14) | Edetate | Appropriate amount |

Preparation method:
(1)-(9) are mixed and dissolved at room temperature to prepare the oil phase beforehand. (10) is added and dispersed and mixed with a disper. (11)-(14) are mixed and dissolved and then gradually added to the oil phase as they are stirred with a disper, followed by thorough mixing and dissolution to obtain the target sunscreen emulsion.

| Example 2-10: Sunscreen emulsion (W/O) | | wt% |
|---|---|---|
| (1) | Decamethylcyclopentasiloxane | 20 |
| (2) | Polyoxyethylene-methylpolysiloxane copolymer | 1 |
| (3) | Olefin oligomer | 10 |
| (4) | Dimethylpolysiloxane | 10 |
| (5) | 2-ethylhexyl p-methoxycinnnamate | 10 |
| (6) | Hexyl diethylaminohydroxybenzoylbenzoate | 5 |
| (7) | 2,4-bis {[4-(2-ethylhexyloxy)-2-hydroxy] phenyll-6-(4-methoxyphenyl)-1,3,5-triazine | 0.5 |
| (8) | Perfume | 0.5 |
| (9) | Dimethicodiethyl benzal malonate | 0.1 |
| (10) | Hydrophobicized titanium oxide | 3 |
| (11) | Paraben | 5 |
| (12) | Ion-exchanged water | Balance |
| (13) | Glycerin | 3 |
| (14) | Edetate | Appropriate amount |

Preparation method:
(1)-(9) are mixed and dissolved at room temperature to prepare the oil phase beforehand. (10) is added and dispersed and mixed with a disper. (11)-(14) are mixed and dissolved and then gradually added to the oil phase as they are stirred with a disper, followed by thorough mixing and dissolution to obtain the target sunscreen emulsion.

| Example 2-11: Sunscreen emulsion (0/W) | | wt% |
|---|---|---|
| (1) | Carboxyvinyl polymer | 0.3 |
| (2) | Purified water | Balance |
| (3) | Xanthan gum | 0.1 |
| (4) | Trisodium edentate | 0.1 |
| (5) | Dipotassium glycyrrhizate | 0.05 |
| (6) | 1,3-butylene glycol | 5 |
| (7) | Polyoxyethylene glyceryl isostearate | 1.5 |
| (8) | Polyoxyethylene glycerol monostearate | 1 |
| (9) | Ascorbic acid glucoside | 2 |
| (10) | Phenoxyethanol | Appropriate amount |
| (11) | Decamethylcyclopentasiloxane | 3 |
| (12) | Methylphenylsiloxane | 3 |
| (13) | Behenyl alcohol | 1 |
| (14) | 2-ethylhexyl p-methoxycinnnamate | 7 |
| (15) | Hexyl diethylaminohydroxybenzoylbenzoate | 2 |
| (16) | 2,4-bis {[4-(2-ethylhexyloxy)-2-hydroxy] phenyl}-6-(4-methoxyphenyl)-1,3,5-triazine | 2 |
| (17) | Dimethicodiethyl benzal malonate | 5 |
| (18) | Perfume | Appropriate amount |
| (19) | Hydrophobicized titanium oxide | 3 |

Preparation method:
(1)-(10) are heated up to 70° C, mixed, and dissolved to prepare the water phase. (11)-(18) are heated, mixed, and dissolved to prepare the oil phase; (19) is dispersed with a disper; the mixture is then gradually added to the water phase as it is stirred with a disper, followed by thorough mixing and dissolution to obtain the target sunscreen cream.

| Example 2-12: Sunscreen cream (W/O) | | wt% |
|---|---|---|
| (1) | Decamethylcyclopentasiloxane | 20 |
| (2) | Polyoxyethylene-methylpolysiloxane copolymer | 3 |
| (3) | Cetyl isooctanoate | 10 |
| (4) | Dimethylpolysiloxane | 5 |
| (5) | 2-ethylhexyl p-methoxycinnnamate | 5 |
| (6) | Hexyl diethylaminohydroxybenzoylbenzoate | 1 |
| (7) | 2,4-bis {[4-(2-ethylhexyloxy)-2-hydroxy] phenyl}-6-(4-methoxyphenyl)-1,3,5-triazine | 3 |
| (8) | Dimethicodiethyl benzal malonate | 3 |
| (9) | 0ctocrylene | 3 |
| (10) | Distearyl ammonium chloride | 0.001 |
| (11) | Phenoxy ethanol | 0.5 |
| (12) | Perfume | 0.5 |
| (13) | Organic modified clay mineral | 2.5 |
| (14) | Hydrophobicized zinc oxide | 5 |
| (15) | Silica powder | 6 |
| (16) | Ion-exchanged water | Balance |
| (17) | 1,3-butylene glycol | 2 |
| (18) | Edetate | Appropriate amount |

Preparation method:
(1)-(12) are heated up to 70° C, mixed and dissolved to prepare the oil phase beforehand. (13)-(15) are added and dispersed and mixed with a disper. (16)-(18) are mixed and dissolved and then gradually added to the oil phase as they are stirred with a disper, followed by thorough mixing and dissolution to obtain the target sunscreen cream.

### INDUSTRIAL APPLICABILITY

According to the present invention, a sunscreen cosmetic that gives a superior sensation during use and manifests a superior ultraviolet absorption effect, as well as a high SPF, can be provided. The present invention is particularly useful as a water-in-oil sunscreen cosmetic.

## Claims

1. A sunscreen cosmetic characteristically comprising (1) 2-ethylhexyl p-methoxycinnnamate, (2) Hexyl diethylaminohydroxybenzoylbenzoate, (3) dimethicodiethyl benzal malonate,
and (4) 2, 4-bis{[4-(2-ethylhexyloxy)-2-hydroxy]phenyl}-6-(4-methoxyphenyl)-1, 3, 5-txiazine.

2. The sunscreen cosmetic of claim 1 wherein said sunscreen cosmetic does not contain an ultraviolet scattering agent.

3. The sunscreen cosmetic of claim 1 or 2
wherein the blend ratio of said (1) 2-ethylhexyl p-methoxycinnnamate is 1-10 wt%, the blend ratio of said (2) Hexyl diethylaminohydroxybenzoylbenzoate is 0.1-10 wt%, the blend ratio of said (3) dimethicodiethyl benzal malonate is 1-10 wt%, and the blend ratio of said (4) 2 ,4-bis{[4-(2-ethylhexyloxy)-2-hydroxy]phenyl}-6-(4-methoxyphenyl)-(1, 3, 5)-triazine is 0.05-3 wt% of the total amount of the sunscreen cosmetic.

4. The sunscreen cosmetic of one of claims 1-3
wherein said sunscreen cosmetic is a water-in-oil emulsified cosmetic.

5. A sunscreen cosmetic characteristically comprising (1) 2-ethylhexyl p-methoxycinnnamate, (2) Hexyl diethylaminohydroxybenzoylbenzoate, (3) dimethicodiethyl benzal malonate, (4) 2, 4-bis{[4-(2-ethylhexyloxy)-2-hydroxy]phenyl}-6-(4-methoxyphenyl)1, 3, 5-triazine, and (5) powder.

6. The sunscreen cosmetic of claim 5 wherein said (5) powder is an ultraviolet scattering agent.

7. The sunscreen cosmetic of claim 6 wherein said ultraviolet scattering agent is titanium oxide and/or zinc oxide.

8. The sunscreen cosmetic of one of claims 5-7
wherein said powder is 0.1-5.0 wt%.

9. The sunscreen cosmetic of one of claims 5-8
wherein the blend ratio of said (1) 2-ethylhexyl p-methoxycinnnamate is 1-10 wt%, the blend ratio of said (2) Hexyl drethylaminohydroxybenzoylbenzoate is 0.1-10 wt%, the blend ratio of said (3) dimethicodiethyl benzal malonate is 1-10 wt%, and the blend ratio of said (4) 2, 4-bis{[4-(2-ethylhexyloxy)-2-hydroxy]phenyl}-6-(4-methoxyphenyl)-(1, 3, 5)-triazine is 0.05-3 wt% of the total amount of the sunscreen cosmetic.

10. The sunscreen cosmetic of one of claims 5-9 wherein said sunscreen cosmetic is a water-in-oil emulsified cosmetic.
